(19) Europäisches Patentamt
European Patent Office
Office européen des brevets

(11) Veröffentlichungsnummer: **0 202 611**
**B1**

(12) # EUROPÄISCHE PATENTSCHRIFT

(45) Veröffentlichungstag der Patentschrift: **15.02.89**

(51) Int. Cl.⁴: **C07D 251/54**

(21) Anmeldenummer: **86106634.8**

(22) Anmeldetag: **15.05.86**

(54) **Verwendung von Lösungsmitteln bei der Herstellung von s-Triazinderivaten und Verfahren zur Herstellung von s-Triazinderivaten.**

(30) Priorität: **24.05.85 DE 3518670**

(43) Veröffentlichungstag der Anmeldung: **26.11.86 Patentblatt 86/48**

(45) Bekanntmachung des Hinweises auf die Patenterteilung: **15.02.89 Patentblatt 89/7**

(84) Benannte Vertragsstaaten: **AT BE CH DE FR GB IT LI NL**

(56) Entgegenhaltungen:
**DE-A- 2 704 067**
**DE-A- 3 206 398**
**DE-A- 3 408 406**

**CHEMICAL ABSTRACTS, Band 96, Nr. 16, 19. April 1982, Columbus, Ohio, USA, KAWAKEN FINE CHEMICALS CO., LTD., TAIYO KORYO K.K., "Water-in-oil emulsifiers and emulsions", p. 446, Zusammenfassung no. 129574b**

(73) Patentinhaber: **BASF Aktiengesellschaft, Carl-Bosch-Strasse 38, D-6700 Ludwigshafen(DE)**

(72) Erfinder: **Martin, Roland, Dr., Bruesseler Ring 53, D-6700 Ludwigshafen(DE)**
Erfinder: **Janitschke, Lothar, Dr., Wormser Gasse 9 a, D-6711 Kleinniedesheim(DE)**

**EP 0 202 611 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft die Verwendung eines speziellen Lösungsmittels bei der Umsetzung von Cyanursäurehalogeniden mit Estern der p-Aminobenzoesäure und ein Verfahren zur technisch einfacheren Herstellung von s-Triazinverbindungen, die als Lichtschutzmittel verwendet werden können.

In der DE-A1 3 206 398 werden s-Triazinderivate, die durch Reste von p-Aminobenzoesäureestern substituiert sind, beschrieben, die außerordentlich gute Lichtschutzmittel darstellen. Gemäß den Herstellungsbeispielen werden die Verbindungen durch Umsetzung von Cyanursäurechlorid mit einem p-Aminobenzoesäureester in verhältnismäßig großen Mengen eines aliphatischen oder aromatischen Kohlenwasserstoffs, insbesondere Benzin oder Xylol, als Lösungsmittel hergestellt. Da aus diesen Lösungsmitteln die gewünschten Verbindungen nur sehr feinkristallin anfallen, entstehen erhebliche technische Probleme bei der abschließenden Filtration, welche unter Umständen mehrere Tage in Anspruch nimmt. Außerdem müssen die verbleibenden Lösungsmittelreste im Endprodukt, ca. 20 bis 30 Gew.-%, durch vorsichtiges Trocknen unter vermindertem Druck vollständig entfernt werden. Diese Trocknung kostet Energie und ist sehr zeitaufwendig, denn wenn die Verbindungen zu schnell bei zu hohen Temperaturen getrocknet werden, weist das anfallende reine Produkt eine geringere Löslichkeit in den in der Kosmetik üblicherweise verwendeten Ölen auf.

Aufgabe der vorliegenden Erfindung ist es, ein technisch möglichst einfaches Herstellungsverfahren für die genannten s-Triazinderivate zu entwickeln, nachdem die gewünschten Lichtschutzmittel großtechnisch in hoher Reinheit und in mindestens gleicher oder noch höherer Ausbeute erhalten werden, wobei die zeitraubende Filtration und Entfernung von Kohlenwasserstoffresten möglichst vermieden werden.

Die Lösung der Aufgabe besteht in der Verwendung von Estern aus einer verzweigten Alkansäure mit 6 bis 10 C-Atomen und einem gesättigten aliphatischen Alkohol mit 10 bis 20 C-Atomen, gegebenenfalls in Form eines Gemischs, als Lösungsmittel bei der Herstellung von s-Triazinderivaten durch Umsetzung von einem Cyanursäurehalogenid mit einem p-Aminobenzoesäureester, der als Rest des Esteralkohols einen Alkylrest mit 6 bis 12 C-Atomen enthält, wie in den Ansprüchen definiert wird.

Als verzweigte Alkansäuren, die den als Lösungsmittel verwendeten Estern zugrundeliegen, kommen solche mit 6 bis 10 C-Atomen in Betracht. Beispielsweise können genannt werden 3,5,5-Trimethylpentansäure oder 3,5,5-Trimethylhexansäure (Isononansäure), und besonders bevorzugt ist die 2-Ethylhexansäure.

Als Esteralkohole kommen gesättigte, geradkettige oder verzweigte aliphatische Alkohole mit 10 bis 20, bevorzugt 12 bis 18 C-Atomen natürlicher oder synthetischer Herkunft in Betracht. Insbesondere sind Fettalkohole, wie Laurylalkohol, Palmitylalkohol, Cetylalkohol oder Stearylalkohol zu nennen. Dabei kann es sich auch um natürliche Gemische oder von der Synthese her um technische Gemische, insbesondere von Alkoholen mit 12 bis 14, 12 bis 18, 16 bis 18 oder 16 bis 20 C-Atomen handeln.

Als erfindungsgemäß zu verwendendes Lösungsmittel ist die 2-Ethylhexansäure verestert mit Cetylalkohol oder Stearylalkohol oder einem Gemisch von Cetyl- und Stearylalkohol, ganz besonders bevorzugt.

Als Cyanursäurehalogenid sind das Cyanursäurebromid und bevorzugt das Cyanursäurechlorid zu nennen.

Die p-Aminobenzoesäureester werden in den 3-fachen molaren Mengen im Verhältnis zum Cyanursäurehalogenid eingesetzt.

Als p-Aminobenzoesäureester sind zu nennen die Ester der p-Aminobenzoesäure mit einem gesättigten, geradkettigen oder verzweigten aliphatischen Alkohol mit 6 bis 12, bevorzugt 8 bis 10 C-Atomen.

Im einzelnen seien beispielsweise genannt der 2-Ethylhexyl-, n-Hexyl-, 3,5,5-Trimethylpentyl-, 3,5,5-Trimethylhexyl-, n-Octyl-, Tetrahydrogeranyl-, n-Decyl-und n-Dodecyl-Ester der p-Aminobenzoesäure.

Die Menge an dem erfindungsgemäß zu verwendenden Lösungsmittel beträgt 30 bis 60 Gew.-%, bezogen auf das Gewicht der Ausgangsverbindungen.

Die Umsetzung erfolgt bei höheren Temperaturen in einem Bereich von 100 bis 250°C, bevorzugt 130 bis 200°C.

Der bei der Reaktion entstehende Chlorwasserstoff wird vorteilhaft mittels eines Stickstoffstromes aus der Lösung entfernt. In der Regel wird nach Beendigung der Reaktion, die mit Hilfe der Dünnschichtchromatographie leicht verfolgt werden kann, noch 1 bis 2 Stunden lang Wasserdampf durch die Reaktionslösung bei Temperaturen von 150 bis 180°C geleitet, um Reste von Salzsäure sowie nichtumgesetzten Cyanursäurehalogenids und eventuell entstehender Nebenprodukte vollständig zu entfernen. Das erhaltene Gemisch enthält das gewünschte s-Triazinderivat in reiner Form. Es erstarrt zu einer glasartigen Masse, die sich zur Weiterverarbeitung in vorteilhafter Weise leicht pulverisieren läßt.

Gegenstand der Erfindung ist auch ein Verfahren zur Herstellung von s-Triazinderivaten der Formel (I)

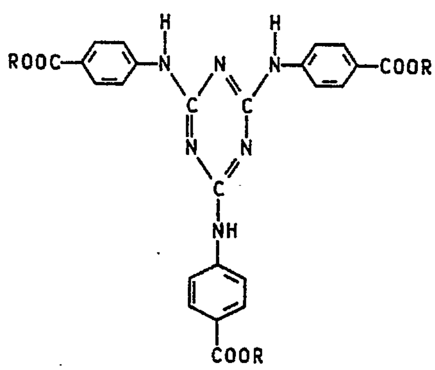

I,

in der R einen Alkylrest mit 6 bis 12 C-Atomen bedeutet, durch Umsetzung eines Cyanursäurehalogenids mit einem p-Aminobenzoesäureester, der als Rest des Esteralkohols einen Alkylrest mit 6 bis 12 C-Atomen enthält, im Molverhältnis 1:3 bei Temperaturen von 100 bis 250°C, dadurch gekennzeichnet, daß man die Umsetzung in einem Ester aus einer verzweigten Alkansäure mit 6 bis 10 C-Atomen und einem gesättigten aliphatischen Alkohol mit 10 bis 20 C-Atomen, gegebenenfalls in Form eines Gemisches, als Lösungsmittel umsetzt.

Das erhaltene Verfahrensprodukt ist besonders geeignet zur Verwendung in kosmetischen Zubereitungen, insbesondere Lichtschutzmitteln. Die besonderen Vorteile der erfindungsgemäßen Verwendung des speziellen Lösungsmittels bzw. des erfindungsgemäßen Verfahrens liegen im Wegfall der aufwendigen Filtration. Ein weiterer überraschender Vorteil ist die große Ausbeutesteigerung bei den p-Aminobenzoesäureestern mit 6 bis 12 C-Atomen von durchweg auf über 90% gegenüber den schwankenden Ausbeuten von 50 bis 80% gemäß der DE-A1 3 206 398 sowie die hohe Reinheit, mit der die Endprodukte anfallen, so daß eine weitere Reinigung nicht erforderlich ist. Die erfindungsgemäß zu verwendenden Lösungsmittel sind Ester, die selbst als kosmetische Öle verwendet werden können. Sie brauchen also nach der Reaktion nicht entfernt zu werden. Die erhaltenen s-Triazinderivate der Formel (I) mit einem Gehalt von 25 bis 45 Gew.-% des erfindungsgemäß zu verwendenden Lösungsmittels können pulverisiert und gesiebt werden. Der Gehalt von 25 bis 45 Gew.-% ergibt sich aus dem eliminierten Halogenwasserstoff und dadurch, daß durch die Wasserdampfdestillation nicht umgesetztes Ausgangsmaterial vollständig entfernt werden kann.

Beispiel 1

1,3,5-Trianilino-p-(carbo-2-ethylhexyl-1-oxy)-s-triazin

737 g (3,0 Mol) p-Aminobenzoesäure-2-ethylhexylester und 400 g eines Esters aus 2-Ethylhexansäure und einem Gemisch aus Cetyl- und Stearylalkohol werden gemischt und auf 100°C erhitzt. Zu der entstandenen Lösung werden 184,4 g (1,0 Mol) Cyanursäurechlorid gegeben und langsam unter Rühren weiter aufgeheizt. Bei ca. 130°C beginnt die Chlorwasserstoffentwicklung. Nach dem Abklingen der ersten Reaktion wird auf eine Innentemperatur von 170°C weiter aufgeheizt. Es entsteht dabei eine Suspension, die im Verlaufe der Nachreaktionszeit von 24 Stunden in eine klare Lösung übergeht. Durch einen leichten Stickstoffstrom wird der bei der Reaktion entstehende Chlorwasserstoff ausgetrieben. Ist die Reaktion beendet (DC-Kontrolle) wird 1 bis 2 Stunden lang Wasserdampf bei einer Temperatur von 170°C durch die Lösung geleitet, um Spuren von Chlorwasserstoff und nicht umgesetztes Ausgangsmaterial zu entfernen. Danach wird unter Rühren auf ca. 130°C abgekühlt und der flüssige Kolbeninhalt abgelassen, wobei er zu einer glasartigen Masse erstarrt. Nach dem Zerkleinern erhält man 1192 g feines Pulver mit einem Gehalt von 63,5% (HPLC-Bestimmung) an 1,3,5-Trianilino-p-(carbo-2-ethylhexyl-1-oxy)-s-triazin, was einer Ausbeute von 92% der Theorie entspricht.

Beispiel 2

1,3,5-Trianilino-p-(carbo-3,7-dimethyloctyl-1-oxy)-s-triazin

81,3 g (0,3 Mol) p-Aminobenzoesäure-3,7-dimethyloctylester, 40 g des Esters aus 2-Ethylhexansäure und einem Gemisch aus Cetyl- und Stearylalkohol und 18,4 g (0,1 Mol) Cyanursäurechlorid werden miteinander gemischt und erhitzt. Bei ca. 110°C beginnt die Chlorwasserstoffentwicklung. Nach dem Abklingen der ersten Reaktion wird weiter auf 180°C aufgeheizt und 42 Stunden bei dieser Temperatur gerührt. Dabei bildet sich eine klare Lösung. Nach dem Ende der Reaktion (DC-Kontrolle) wird noch 2 Stunden Wasserdampf durch die Reaktionsmischung bei Temperaturen von 180°C geleitet. Es wird auf 100°C abgekühlt und der flüssige Kolbeninhalt abgelassen. Beim weiteren Abkühlen erstarrt die Masse glasartig. Man erhält 120 g eines Pulvers mit einem Gehalt von 65% (HPLC-Bestimmung) an 1,3,5-Trianilino-p-(carbo-3,7-dimethyloctyl-1-oxy)-s-triazin, was einer Ausbeute von 86% der Theorie entspricht.

## Patentansprüche

1. Verwendung von Estern aus einer verzweigten Alkansäure mit 6 bis 10 C-Atomen und einem gesättigten aliphatischen Alkohol mit 10 bis 20 C-Atomen, gegebenenfalls in Form eines Gemisches, als Lösungsmittel bei der Herstellung von s-Triazinderivaten der Formel (I)

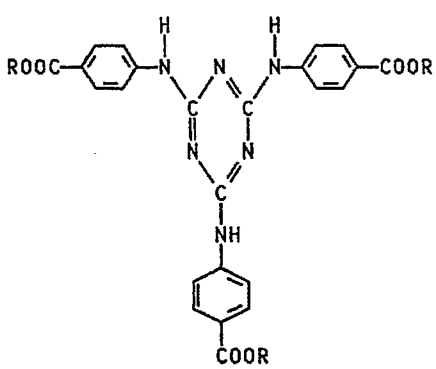

I

in der R einen Alkylrest von 6 bis 12 C-Atomen bedeutet, durch Umsetzung eines Cyanursäurehalogenids mit einem p-Aminobenzoesäureester, der als Rest des Esteralkohols einen Alkylrest mit 6 bis 12 C-Atomen enthält, im Molverhältnis 1:3 bei Temperaturen von 100 bis 250°C.

2. Verwendung von Estern als Lösungsmittel gemäß Anspruch 1 in einer Menge von 30 bis 60 Gew.-%, bezogen auf das Gesamtgewicht der Ausgangsverbindungen.

3. Verwendung von 2-Ethylhexansäure, verestert mit Cetyl- oder Stearylalkohol oder ihres Gemisches, als Lösungsmittel nach Anspruch 1 oder 2.

4. Verfahren zur Herstellung von s-Triazinderivaten der Formel (I)

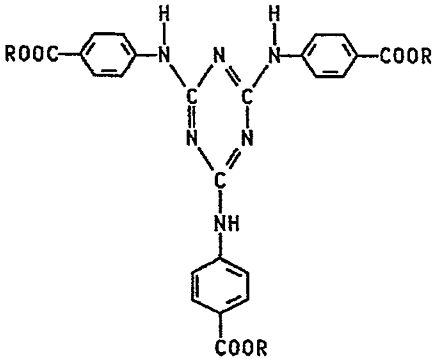

I

in der R einen Alkylrest von 6 bis 12 C-Atomen bedeutet, durch Umsetzung eines Cyanursäurehalogenids mit einem p-Aminobenzoesäureester, der als Rest des Esteralkohols einen Alkylrest mit 6 bis 12 C-Atomen enthält, im Molverhältnis 1:3 bei Temperaturen von 100 bis 250°C, dadurch gekennzeichnet, daß man die Umsetzung in einem Ester aus einer verzweigten Alkansäure mit 6 bis 10 C-Atomen und einem gesättigten aliphatischen Alkohol mit 10 bis 20 C-Atomen, gegebenenfalls in Form eines Gemischs, als Lösungsmittel durchfährt.

5. Verfahren nach Anspruch 4, dadurch gekennzeichnet, daß die Umsetzung in einer Menge an Lösungsmittel von 30 bis 60 Gew.-%, bezogen auf das Gesamtgewicht der Ausgangsverbindungen, durchgeführt wird.

6. Verfahren nach Anspruch 4 oder 5, dadurch gekennzeichnet, daß die Umsetzung in 2-Ethylhexansäure verestert mit Cetyl- oder Stearylalkohol oder ihres Gemisches, als Lösungsmittel durchgeführt wird.

## Claims

1. Use of an ester of a branched alkanoic acid of 6 to 10 carbon atoms with a saturated aliphatic alcohol of 10 to 20 carbon atoms, or with, if appropriate, a mixture of such alcohols, as the solvent in the preparation of an s-triazine deviation of the formula (I)

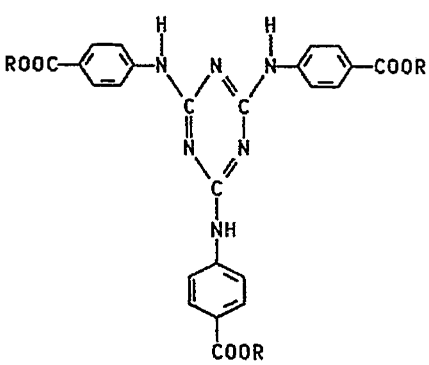

where R is alkyl of 6 to 12 carbon atoms, by reacting a cyanuric halide with a p-aminobenzoic ester which contains, as the ester alcohol radical, alkyl of 6 to 12 carbon atoms, in a molar ratio of 1:3 at from 100 to 250°C.

2. Use of an ester solvent as claimed in claim 1 in from 30 to 60% by weight based on the total weight of the starting compounds.

3. Use of 2-ethylhexanoic acid esterified with cetyl or stearyl alcohol, or with a mixture thereof, as a solvent as set forth in claim 1 or 2.

4. A process for the preparation of an s-triazine derivative of the formula (I)

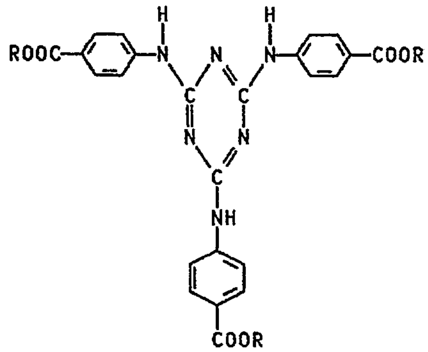

where R is alkyl of 6 to 12 carbon atoms, by reacting a cyanuric halide with a p-aminobenzoic ester which contains, as the ester alcohol radical, alkyl of 6 to 12 carbon atoms in a molar ratio of 1:3 at from 100 to 250°C which comprises performing the reaction in a solvent comprising an ester of a branched alkanoic acid of 6 to 10 carbon atoms with a saturated aliphatic alcohol of 10 to 20 carbon atoms or with, if appropriate, a mixture of such alcohols.

5. A process as claimed in claim 4, wherein the reaction is carried out in from 30 to 60% by weight, based on the total weight of the starting compounds, of solvent.

6. A process as claimed in claim 4 or 5, wherein the reaction is carried out in, as the solvent, 2-ethylhexanoic acid esterified with cetyl or stearyl alcohol or with a mixture thereof.

## Revendications

1. Utilisation d'esters d'un acide alcanecarboxylique ramifié à 6–10 atomes de carbone et d'un alcool aliphatique saturé à 10–20 atomes de carbone, éventuellement sous forme d'un mélange, comme solvant pour la préparation de dérivés de s-triazine de formule (I)

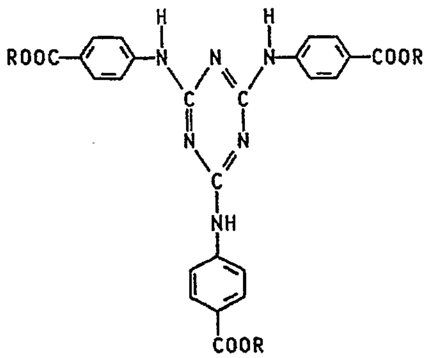

I

dans laquelle R est un reste alkyle à 6–12 atomes de carbone, par réaction, à une température de 100 à 250°C et dans un rapport molaire de 1:3, d'un halogénure d'acide cyanurique avec un ester d'acide p-aminobenzoïque qui contient comme reste de l'alcool formant l'ester un reste alkyle à 6–12 atomes de carbone.

2. Utilisation d'esters comme solvant selon la revendication 1, en quantité comprise entre 30 et 60% en poids par rapport au poids total des substances de départ.

3. Utilisation d'acide 2-éthylhexanoïque estérifié par l'alcool cétylique ou stéarylique ou leurs mélanges, comme solvant, selon la revendication 1 ou 2.

4. Procédé de préparation de dérivés de s-triazine de formule (I)

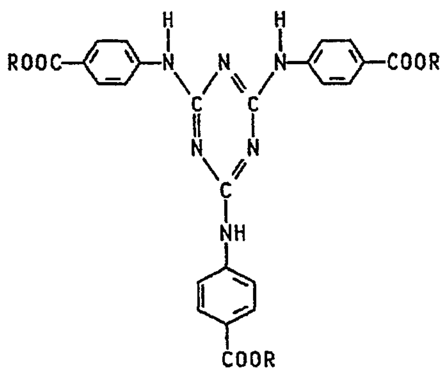

I

dans laquelle R est un reste alkyle à 6–12 atomes de carbone, par reaction, à une température de 100 à 250°C et dans un rapport molaire de 1:3, d'un halogénure d'acide cyanurique avec un ester d'acide p-aminobenzoïque qui contient comme reste de l'alcool formant l'ester un reste alkyle à 6–12 atomes de carbone, caractérisé en ce qu'on mène la réaction dans un solvant qui est un ester d'un acide alcanecarboxylique ramifié à 6–10 atomes de carbone et d'un alcool aliphatique saturé à 10–20 atomes de carbone, éventuellement sous forme d'un mélange.

5. Procédé selon la revendication 4, caractérisé en ce que la réaction est menée dans une quantité de solvant de 30 à 60% en poids par rapport au poids total des substances de départ.

6. Procédé selon la revendication 4 ou 5, caractérisé en ce que la réaction est menée dans un solvant qui est de l'acide 2-éthylhexanoïque estérifié par l'alcool cétylique ou stéarylique ou leurs mélanges.